(19) **Europäisches Patentamt / European Patent Office / Office européen des brevets**

(11) **EP 4 052 791 A1**

(12) **EUROPEAN PATENT APPLICATION**
published in accordance with Art. 153(4) EPC

(43) Date of publication:
**07.09.2022 Bulletin 2022/36**

(21) Application number: **21861937.7**

(22) Date of filing: **10.08.2021**

(51) International Patent Classification (IPC):
$B01J\ 37/08^{(2006.01)}$   $B01J\ 37/04^{(2006.01)}$
$B01J\ 23/10^{(2006.01)}$   $B01J\ 21/06^{(2006.01)}$
$C01G\ 25/02^{(2006.01)}$   $C04B\ 35/48^{(2006.01)}$
$C04B\ 35/50^{(2006.01)}$   $C07C\ 5/333^{(2006.01)}$
$C07C\ 11/167^{(2006.01)}$

(52) Cooperative Patent Classification (CPC):
**B01J 21/06; B01J 23/10; B01J 37/04; B01J 37/08;
C01G 25/02; C04B 35/48; C04B 35/50;
C07C 5/333; C07C 11/167**

(86) International application number:
**PCT/KR2021/010534**

(87) International publication number:
**WO 2022/045640 (03.03.2022 Gazette 2022/09)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **31.08.2020 KR 20200110340**

(71) Applicant: **Lg Chem, Ltd.**
**Seoul 07336 (KR)**

(72) Inventors:
• **HAN, Sang Jin**
**Daejeon 34122 (KR)**

• **KO, Dong Hyun**
**Daejeon 34122 (KR)**
• **KANG, Jun Han**
**Daejeon 34122 (KR)**
• **HWANG, Ye Seul**
**Daejeon 34122 (KR)**
• **HWANG, Sunhwan**
**Daejeon 34122 (KR)**

(74) Representative: **Goddar, Heinz J.**
**Boehmert & Boehmert
Anwaltspartnerschaft mbB
Pettenkoferstrasse 22
80336 München (DE)**

(54) **METHOD FOR PREPARING CERIA-ZIRCONIA COMPOSITE OXIDE, CERIA-ZIRCONIA COMPOSITE OXIDE, CATALYST COMPRISING SAME, AND METHOD FOR PREPARING BUTADIENE**

(57)    The present disclosure relates to a method for preparing a ceria-zirconia composite oxide, a ceria-zirconia composite oxide, and a catalyst including the same.

**EP 4 052 791 A1**

**(Cont. next page)**

【FIG. 1】

**EP 4 052 791 A1**

**Description**

[Technical Field]

This application claims priority to and the benefits of Korean Patent Application No. 10-2020-0110340, filed with the Korean Intellectual Property Office on August 31, 2020, the entire contents of which are incorporated herein by reference.

This application relates to a method for preparing a ceria-zirconia composite oxide, a ceria-zirconia composite oxide, a catalyst including the same, and a method for preparing butadiene.

[Background Art]

A reaction using a ferrite-based catalyst is mostly an exothermic reaction generating heat, and produces a large amount of heavy substances. In order to remove these, a ceria-zirconia composite oxide needs to be used in the reaction using a ferrite-based catalyst.

The present disclosure relates to a method for preparing a ceria-zirconia composite oxide, a ceria-zirconia composite oxide, a catalyst including the same, and a method for preparing butadiene using the same.

[Disclosure]

[Technical Problem]

As described above, a reaction using a ferrite-based catalyst is mostly an exothermic reaction generating heat, and produces a large amount of heavy substances. In order to remove these, a ceria-zirconia composite oxide needs to be used in the reaction using a ferrite-based catalyst.

The present disclosure relates to a method for preparing a ceria-zirconia composite oxide, a ceria-zirconia composite oxide, a catalyst including the same, and a method for preparing butadiene using the same.

[Technical Solution]

One embodiment of the present disclosure provides a method for preparing a ceria-zirconia composite oxide, the method including preparing an aqueous precursor solution by mixing a cerium precursor compound and a zirconia precursor compound; stirring the aqueous precursor solution under a temperature condition of 30°C to 120°C; and calcining the result.

In addition, one embodiment of the present disclosure provides a ceria-zirconia composite oxide prepared using the above-described method, and including cerium (Ce) and zirconia (Zr) in a molar ratio of 1:2 to 20:1.

In addition, one embodiment of the present disclosure provides a catalyst including the ceria-zirconia composite oxide described above and a zinc-ferrite-based catalyst.

In addition, one embodiment of the present disclosure provides a method for preparing butadiene, the method including forming a catalyst layer by filling a reactor with the above-described catalyst; and inducing an oxidative dehydrogenation reaction while continuously passing a reactant including a C4 fraction and oxygen through the catalyst layer.

[Advantageous Effects]

A preparation method according to one embodiment of the present disclosure readily forms crystals of a ceria-zirconia composite oxide, and, when used in a reaction using a ferrite-based catalyst, is effective in reducing the reaction temperature and heavy substances.

[Description of Drawings]

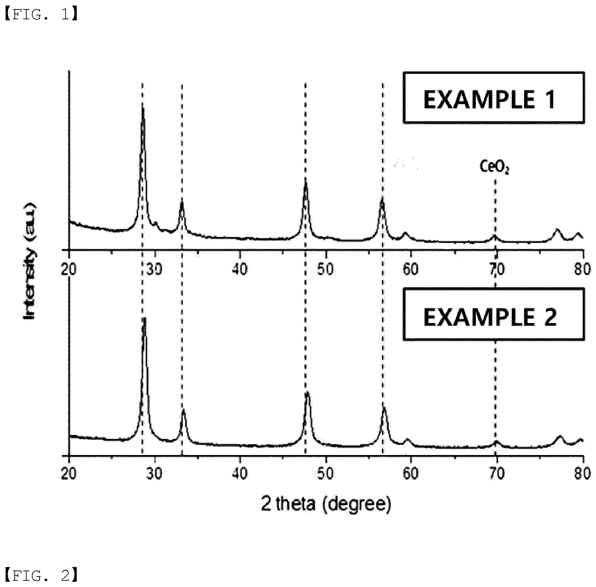

FIG. 1 to FIG. 3 are XRD diagrams showing results of Experimental Example 1.

[Mode for Disclosure]

Hereinafter, specific embodiments for working of the present disclosure will be described.

In the present specification, "water" may be distilled water (DI water).

In the present specification, a 'yield (%)' is defined as a value obtained by dividing the number of moles of 1,3-butadiene, a product of an oxidative dehydrogenation reaction, by the number of moles of butene, a raw material. For example, the yield may be expressed by the following equation.

$$Yield\ (\%)=[(number\ of\ moles\ of\ produced\ 1,3{-}butadiene)/(number\ of\ moles\ of\ supplied\ butene)]X100$$

In the present specification, a 'conversion ratio (%)' refers to a ratio of a reactant being converted to a product, and for example, the conversion ratio of butene may be defined by the following equation.

$$Conversion\ ratio\ (\%)=[(number\ of\ moles\ of\ reacted\ butene)/(number\ of\ moles\ of\ supplied\ butene)]X100$$

In the present specification, 'selectivity (%)' is defined as a value obtained by dividing an amount of change in butadiene (BD) by an amount of change in butene (BE). For example, the selectivity may be expressed by the following equation.

$$Selectivity\ (\%)=[(number\ of\ moles\ of\ produced\ 1,3{-}butadiene\ or\ number\ of\ moles\ of\ COx)/(number\ of\ moles\ of\ reacted\ butene)]\ X\ 100$$

One embodiment of the present disclosure provides a method for preparing a ceria-zirconia composite oxide, the method including preparing an aqueous precursor solution by mixing a cerium precursor compound and a zirconia precursor compound; stirring the aqueous precursor solution under a temperature condition of 30°C to 120°C; and calcining the result.

The ceria-zirconia composite oxide is used to remove heavy substances generated in a reaction using a ferrite-based catalyst. Herein, performance of the ceria-zirconia composite oxide is affected by a crystal structure of the composite oxide. The present disclosure relates to a method for preparing a ceria-zirconia composite oxide, and enhances crystallinity of the ceria-zirconia composite oxide by treating at a high temperature of 30°C to 120°C when stirring the aqueous precursor solution.

In the stirring, the temperature condition may be adjusted to 60°C to 120°C, and preferably 80°C to 100°C. When satisfying the above-mentioned numerical range, crystallinity of the ceria-zirconia composite oxide may increase. When the temperature condition is less than the above-mentioned range, crystallinity of the composite oxide may decrease, and when the temperature condition is greater than the above-mentioned range, denaturation may occur in the stirring. The temperature may be adjusted by conducting the stirring in an oven capable of stirring, and for example, a hot plate or a dual jacket may be used.

In one embodiment of the present disclosure, the stirring may be conducted for 0.1 h to 10 h, 1 h to 8 h, or 1 h to 2 h. When the stirring time is less than the above-mentioned range, it is difficult to sufficiently volatize the solvent included in the aqueous solution, and therefore, stirring for the above-mentioned time is required. The h means hours.

In one embodiment of the present disclosure, the aqueous precursor solution may include any one or more selected from among nitric acid, hydrochloric acid, acetic acid, ammonia water, alcohol and mixture solutions thereof. This has an advantage of favorably combining cerium and zirconia and increasing crystallinity compared to when including water alone.

In one embodiment of the present disclosure, the cerium precursor compound may be selected from the group consisting of cerium nitrate, cerium ammonium nitrate, cerium sulfate, cerium ammonium sulfate, cerium alkoxide and mixtures thereof.

In one embodiment of the present disclosure, the zirconia precursor compound may be selected from the group consisting of zirconium carboxylate, zirconium halide, zirconium oxyhalide, zirconium carbonate, zirconium nitrate, zirconium oxynitrate, zirconium sulfate and mixtures thereof.

In one embodiment of the present disclosure, the cerium precursor compound and the zirconia precursor compound may have a molar ratio of 1:2 to 20:1, 1:1 to 10:1, 1:1 to 5:1 or 1:1 to 3:1. When forming the oxide, a structure of the zirconia element surrounding the cerium element is formed since cerium (Ce) has a larger atomic radius than zirconia, and when the zirconia element ratio is greater than the above-mentioned range, it is difficult to form the structure causing a problem of reducing structural stability. Conducting a reaction using such a catalyst causes a problem of increasing a reaction temperature, and therefore, it is preferred to satisfy the above-mentioned range.

In one embodiment of the present disclosure, the aqueous precursor solution may further include a precipitating agent in the stirring. The precipitating agent is typically a basic compound, and may be preferably selected from any suitable basic materials such as alkali and alkaline-earth metal carbonates, ammonium and alkylammonium carbonates, ammonium and alkylammonium hydroxides, alkali and alkaline-earth metal hydroxides, water-soluble organic base compounds and mixtures thereof. The precipitating agent is preferably ammonium hydroxide or sodium hydroxide.

In one embodiment of the present disclosure, the method for preparing a ceria-zirconia composite oxide may further include, before the calcining, one or more steps selected from the group consisting of filtering, washing and spray drying. This is for separating the formed oxide particles before the calcination.

In one embodiment of the present disclosure, the calcining is a step of oxidizing the stirred cerium-zirconia composite.

In one embodiment of the present disclosure, the calcining may be conducted at a temperature of 400°C to 1,000°C, 500°C to 900°C, or 500°C to 700°C. When the temperature is in the above-mentioned range, crystallinity of the calcined composite oxide increases, and when the temperature is outside the above-mentioned range, crystallization may excessively occur in some cases.

In one embodiment of the present disclosure, the calcining may be conducted under the air atmosphere.

In one embodiment of the present disclosure, the calcining may be conducted for 0.5 hours to 24 hours, 1 hour to 20 hours, or 2 hours to 15 hours. When the calcination time is too short, the composite oxide may not be sufficiently oxidized, and when the calcination time is too long, the composite oxide may be excessively oxidized in some cases, and therefore, the calcination time needs to be adjusted to the above-mentioned range.

One embodiment of the present disclosure provides a ceria-zirconia composite oxide prepared using the above-described preparation method and including cerium (Ce) and zirconia (Zr) in a molar ratio of 1:2 to 20:1.

In one embodiment of the present disclosure, the cerium (Ce) and the zirconia (Zr) may have a molar ratio of 1:2 to 20:1, 1:1 to 10:1, 1:1 to 5:1, or 1:1 to 3:1. When forming the oxide, a structure of the zirconia element surrounding the cerium element is formed since cerium (Ce) has a larger atomic radius than zirconia, and when the zirconia element ratio is greater than the above-mentioned range, it is difficult to form the structure causing a problem of reducing structural stability. Conducting a reaction using such a catalyst causes a problem of increasing a reaction temperature, and therefore, it is preferred to satisfy the above-mentioned range.

One embodiment of the present disclosure provides a catalyst including the ceria-zirconia composite oxide described above and a zinc-ferrite-based catalyst.

In one embodiment of the present disclosure, the zinc-ferrite-based catalyst may be supported on the ceria-zirconia composite oxide.

In one embodiment of the present disclosure, a content of the ceria-zirconia composite oxide may be greater than or equal to 0.01 wt% and less than or equal to 0.1 wt%, greater than or equal to 0.01 wt% and less than or equal to 0.08 wt%, or greater than or equal to 0.01 wt% and less than or equal to 0.06 wt% based on a total weight of the zinc-ferrite-based catalyst. The ceria-zirconia composite oxide content

An oxidative dehydrogenation reaction of butene of the present disclosure may be named as a method for preparing butadiene. The method for preparing butadiene may include conducting an oxidative dehydrogenation reaction while passing through a reactant including a C4 fraction and oxygen in a reactor filled with the zinc-ferrite catalyst prepared using the above-described preparation method.

The method for preparing butadiene includes forming a catalyst layer by filling a reactor with the zinc-ferrite catalyst prepared using the above-described preparation method; and inducing an oxidative dehydrogenation reaction while continuously passing a reactant including a C4 fraction and oxygen through the catalyst layer.

The C4 fraction may mean C4 raffinate-1,2,3 remaining after separating useful compounds from a C4 mixture produced by naphtha cracking, and may mean C4 class that may be obtained through ethylene dimerization. Specifically, the C4 fraction may be one, or a mixture of two or more selected from the group consisting of n-butane, trans-2-butene, cis-2-butene and 1-butene.

The reactant may include steam or nitrogen ($N_2$) in addition to the C4 fraction and oxygen. The steam or nitrogen ($N_2$) is a diluent gas introduced for the purposes of preventing catalyst coking, removing reaction heat and the like as well as reducing the risk of reactant explosion.

The oxygen ($O_2$) is an oxidant, and reacts with the C4 fraction to produce a dehydrogenation reaction.

The oxidative dehydrogenation reaction may be conducted according to the following Reaction Formula 1 or Reaction Formula 2.

[Reaction Formula 1] $\quad$ $C_4H_8 + 1/2 O_2 \rightarrow C_4H_6 + H_2O$

[Reaction Formula 2] $\quad$ $C_4H_{10} + O_2 \rightarrow C_4H_6 + 2H_2O$

Butadiene is prepared by removing hydrogen of butane or butene through the oxidative dehydrogenation reaction. Meanwhile, in the oxidative dehydrogenation reaction, side reaction products including carbon monoxide (CO), carbon dioxide ($CO_2$) or the like may be produced in addition to the main reaction such as Reaction Formula 1 or 2. A process of separating and discharging the side reaction products out of the system may be included so that these are not continuously accumulated in the process.

In the method for preparing butadiene, the conversion ratio of butene may be 40% or greater, preferably 45% or greater, and more preferably 50% or greater.

In the method for preparing butadiene, the selectivity of butadiene may be 40% or greater, preferably 45% or greater, and more preferably 50% or greater.

The oxidative dehydrogenation reaction may be reacted under a condition of GHSV (gas hourly space velocity) of 100 $h^{-1}$ to 400 $h^{-1}$ under a condition of a reaction temperature of 200°C to 600°C and a pressure of 0.1 bar to 10 bar.

Hereinafter, the present specification will be described in detail with reference to examples. However, examples according to the present specification may be modified to various other forms, and the scope of the present specification is not to be construed as being limited to the examples described below. The examples of the present specification are provided in order to more fully describe the present specification to those having average knowledge in the art.

**Example**

**Example 1: Preparation of Ceria-Zirconia Composite Oxide**

A cerium precursor compound [$Ce(NO_3)_3*6H_2O$], a zirconia precursor compound [$ZrO(NO_3)_2*2H_2O$] and ammonia water were mixed to prepare an aqueous precursor solution. Herein, the cerium precursor compound and the zirconia precursor compound had a molar ratio of 1:1.

After that, a basic solution was added to the aqueous precursor solution followed by stirring, and, while maintaining the pH at 13 or higher, the result was stirred while heating for 1 hour to 2 hours at a temperature of 90°C.

The filtered material was dried under an air environment for 12 hours at a temperature of 120°C in an oven.

After that, the result was calcined for 6 hours at a temperature of 650°C under an air environment to prepare a final ceria-zirconia composite oxide. Herein, the cerium and the zirconium had an element ratio of 1:1.

**Example 2: Preparation of Ceria-Zirconia Composite Oxide**

A ceria-zirconia composite oxide was prepared in the same manner as in Example 1 except that the element ratio of the cerium and the zirconium was adjusted to 1:0.1.

**Comparative Example 1: Preparation of Ceria-Zirconia Composite Oxide**

A ceria-zirconia composite oxide was prepared in the same manner as in Example 1 except that the aqueous precursor solution was not heated and the temperature was maintained at room temperature (25°C).

**Comparative Example 2: Preparation of Ceria-Zirconia Composite Oxide**

A ceria-zirconia composite oxide was prepared in the same manner as in Example 1 except that the aqueous precursor solution was not heated and the temperature was maintained at room temperature (25°C), and the aqueous precursor solution did not include solvents other than water.

**Experimental Example 1: Identification of Ceria-Zirconia Composite Oxide Synthesis**

Crystal states of the ceria-zirconia composite oxides prepared in the examples and the comparative examples were identified using an X-ray diffraction apparatus (Endeavor XRD apparatus of Bruker).

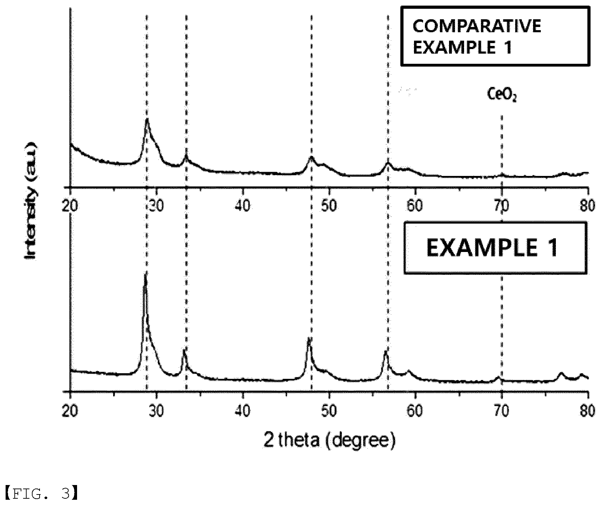

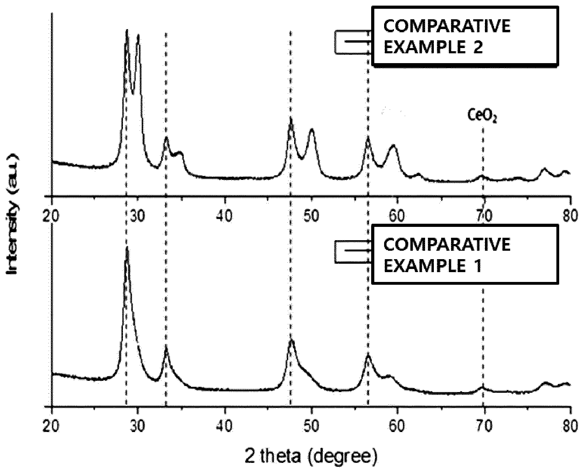

As a result, when the aqueous precursor solution did not include solvents other than water (Comparative Examples 1 and 2), cerium and zirconia were not composited to each other, and only the presence of $ZrO_2$ and $CeO_2$ corresponding to each oxide thereof was identified (FIG. 3).

It was identified that, when the temperature was 90°C during the stirring (Example 1), intensity of the peak corresponding to the ceria-zirconia composite oxide was high compared to when the synthesis was conducted at room temperature (25°C) (Comparative Example 1), and the result is shown in FIG. 2. As a result, it was identified that intensity of the peak corresponding to $ZrO_2$ became weak and the peak corresponding to $CeO_2$ was shifted backward. This is due to the fact that pure $ZrO_2$ penetrated into $CeO_2$ to form crystals.

From the results, it was identified that, in order to favorably form the ceria-zirconia composite oxide, the aqueous precursor solution needs to include solvents such as ammonia water, and the stirring needs to be conducted at a high temperature.

**Experimental Example 2: Experiment on Catalyst Performance Example 3: Preparation of Catalyst Including Ceria-Zirconia Composite Oxide and a Zinc-Ferrite-Based Catalyst**

The ceria-zirconia composite oxide prepared in Example 1 and a zinc-ferrite-based catalyst were mixed with other, and the mixture was mixed with water to prepare catalyst slurry. The zinc-ferrite-based catalyst was prepared as follows. Herein, the zinc-ferrite-based catalyst was mixed in a weight ratio of 0.036 wt% based on the ceria-zirconia composite oxide.

Zinc chloride ($ZnCl_2$) (0.122 mol) and ferric chloride ($FeCl_3 6H_2O$) (0.243 mol) were dissolved in water (12.778 mol) to prepare a metal aqueous precursor solution having a metal precursor concentration of approximately 26.4 wt% in the aqueous solution. Herein, the molar ratio of the metal components included in the metal aqueous precursor solution was Fe:Zn=2:1.

Next, a coprecipitation bath (pH: 7 to 9) prepared with ammonia water was prepared, and a process of coprecipitating iron and zinc was conducted by dropwise adding the metal aqueous precursor solution with ammonia water having a concentration of 9 wt% to 10 wt%. The coprecipitated solution was stirred for 1 hour for sufficient coprecipitation, and after stopping the stirring, the coprecipitates were aged by being left unattended for 1 hour at room temperature (25°C) so that all the precipitates were settled.

The coprecipitated solution completed with the stirring and the aging was vacuum filtered using a vacuum filter to obtain the coprecipitates, and the coprecipitates were washed and then dried for 24 hours at 90°C in an oven under air. The dried coprecipitates were introduced to a furnace with an inner temperature of 80°C, and, after raising the temperature to 650°C at a temperature-raising rate of 1°C/min, heat treated for 6 hours at the same temperature. After that, the temperature was lowered to 20°C to prepare a final zinc-ferrite catalyst.

**Example 4: Preparation of Catalyst Including Ceria-Zirconia Composite Oxide and Zinc-Ferrite-Based Catalyst**

A catalyst was prepared in the same manner as in Example 3 except that the ceria-zirconia composite oxide prepared in Example 2 was used instead of the ceria-zirconia composite oxide prepared in Example 1.

**Comparative Example 3: Preparation of Catalyst Including Ceria-Zirconia Composite Oxide and Zinc-Ferrite-Based Catalyst**

A catalyst was prepared in the same manner as in Example 3 except that the ceria-zirconia composite oxide prepared in Comparative Example 1 was used instead of the ceria-zirconia composite oxide prepared in Example 1.

**Comparative Example 4: Preparation of Catalyst Not Including Ceria-Zirconia Composite Oxide**

A catalyst was prepared in the same manner as in Example 3 except that the ceria-zirconia composite oxide was not included.

The slurry including each of the catalysts prepared in Example 3, Example 4, Comparative Example 3 and Comparative Example 4 was installed as a BED in a reactor, and after conducting an oxidative dehydrogenation reaction, products were analyzed by gas chromatography (GC), and conversion ratio of butene (X_BE), selectivity of butadiene (S_BD), yield of butadiene (Y_BD), selectivity of Cox (S_Cox) and selectivity of heavy substances (S_heavy) are shown in the following Table 1 and Table 2.

Herein, Experiment 1 and Experiment 2 were conducted while varying inlet gas compositions, and OBR, SBR and NBR respectively mean OBR=oxygen/total 2-butene ratio, SBR=steam/total 2-butene ratio and NBR=nitrogen/total 2-butene ratio.

[Table 1]

| Experiment 1: Reaction Condition: OBR/NBR/SBR=0.85/8/1 | | | | | | |
|---|---|---|---|---|---|---|
| Catalyst Type | Process Temperature (°C) | X_BE (%) | S_BD (%) | Y_BD (%) | S_Cox (%) | S_hea vy (%) |
| Example 3 | 356 | 65.1 | 86.7 | 56.4 | 12.6 | 0.8 |
| Comparative Example 3 | 353 | 67.9 | 83.3 | 56.6 | 15.2 | 1.5 |

(continued)

| Experiment 1: Reaction Condition: OBR/NBR/SBR=0.85/8/1 | | | | | | |
|---|---|---|---|---|---|---|
| Catalyst Type | Process Temperature (°C) | X_BE (%) | S_BD (%) | Y_BD (%) | S_Cox (%) | S_hea vy (%) |
| Comparative Example 4 | 366 | 72.9 | 87.9 | 64 | 11.1 | 1 |

[Table 2]

| Experiment 2: Reaction Condition: OBR/NBR/SBR=1/8/1 | | | | | | |
|---|---|---|---|---|---|---|
| Catalyst Type | Process Temperature (°C) | X_BE (%) | S_BD (%) | Y_BD (%) | S_Cox (%) | S_hea vy (%) |
| Example 3 | 331 | 73.2 | 84.3 | 61.7 | 14.6 | 1 |
| Example 4 | 365 | 77.5 | 85.8 | 66.5 | 13.4 | 0.7 |
| Comparative Example 4 | 377 | 79.9 | 84.5 | 67.6 | 14.5 | 0.9 |

From the results, it was identified that, when conducting the oxidative dehydrogenation reaction using the catalyst not including the ceria-zirconia composite oxide as in the art, the process temperature increased by failing to control an exothermic phenomenon (Comparative Example 4 of Table 1 and Table 2).

Meanwhile, even when the catalyst includes the ceria-zirconia composite oxide, it was identified that selectivity of the side reaction materials (Cox, heavy substances) increased as a result of decreasing crystallinity of the oxide when the aqueous precursor solution was stirred at a low temperature (25°C) in the ceria-zirconia composite oxide preparation (Comparative Example 3 of Table 1).

From the results, it was identified that, in order to control an exothermic phenomenon and reduce side reaction materials at the same time during the oxidative dehydrogenation reaction, inside the reactor needs to be filled with a catalyst in which the zinc-ferrite-based catalyst and the ceria-zirconia composite oxide are mixed, and crystallinity of the oxide needs to be increased by stirring at a high temperature during the ceria-zirconia composite oxide preparation.

**Claims**

1. A method for preparing a ceria-zirconia composite oxide, the method comprising:

   preparing an aqueous precursor solution by mixing a cerium precursor compound and a zirconia precursor compound;
   stirring the aqueous precursor solution under a temperature condition of 30°C to 120°C; and
   calcining the result.

2. The method for preparing a ceria-zirconia composite oxide of Claim 1, wherein the stirring is conducted for 0.1 hours to 10 hours.

3. The method for preparing a ceria-zirconia composite oxide of Claim 1, wherein the aqueous precursor solution includes any one or more solvents selected from among nitric acid, hydrochloric acid, acetic acid, ammonia water, alcohol and mixture solvents thereof.

4. The method for preparing a ceria-zirconia composite oxide of Claim 1, wherein the cerium precursor compound is selected from the group consisting of cerium nitrate, cerium ammonium nitrate, cerium sulfate, cerium ammonium sulfate, cerium alkoxide and mixtures thereof.

5. The method for preparing a ceria-zirconia composite oxide of Claim 1, wherein the zirconia precursor compound is selected from the group consisting of zirconium carboxylate, zirconium halide, zirconium oxyhalide, zirconium carbonate, zirconium nitrate, zirconium oxynitrate, zirconium sulfate and mixtures thereof.

6.  The method for preparing a ceria-zirconia composite oxide of Claim 1, wherein the cerium precursor compound and the zirconia precursor compound have a molar ratio of 1:2 to 20:1.

7.  The method for preparing a ceria-zirconia composite oxide of Claim 1, wherein the stirring further includes adding one or more precipitating agents selected from the group consisting of alkali and alkaline-earth metal carbonates, ammonium and alkylammonium carbonates, ammonium and alkylammonium hydroxides, alkali and alkaline-earth metal hydroxides, water-soluble organic base compounds and mixtures thereof.

8.  The method for preparing a ceria-zirconia composite oxide of Claim 1, wherein the calcining is conducted at a temperature of 400°C to 1,000°C.

9.   A ceria-zirconia composite oxide prepared using the preparation method of any one of Claims 1 to 8, and comprising cerium (Ce) and zirconia (Zr) in a molar ratio of 1:2 to 20:1.

10. A catalyst comprising:

    the ceria-zirconia composite oxide of Claim 9; and
    a zinc-ferrite-based catalyst.

11. The catalyst of Claim 10, wherein, based on a total weight of the zinc-ferrite-based catalyst, a content of the ceria-zirconia composite oxide is greater than or equal to 0.01 wt% and less than or equal to 0.1 wt%.

12. A method for preparing butadiene, the method comprising:

    forming a catalyst layer by filling a reactor with the catalyst of Claim 10; and
    inducing an oxidative dehydrogenation reaction while continuously passing a reactant including a C4 fraction and oxygen through the catalyst layer.

13. The method for preparing butadiene of Claim 12, wherein the oxidative dehydrogenation reaction is reacted under a condition of GHSV (gas hourly space velocity) of 100 $h^{-1}$ to 400 $h^{-1}$ under a condition of a reaction temperature of 200°C to 600°C and a pressure of 0.1 bar to 10 bar.

【FIG. 1】

【FIG. 2】

【FIG. 3】

# EP 4 052 791 A1

| INTERNATIONAL SEARCH REPORT | International application No. |
|---|---|
| | **PCT/KR2021/010534** |

**A. CLASSIFICATION OF SUBJECT MATTER**

**B01J 37/08**(2006.01)i; **B01J 37/04**(2006.01)i; **B01J 23/10**(2006.01)i; **B01J 21/06**(2006.01)i; **C01G 25/02**(2006.01)i; **C04B 35/48**(2006.01)i; **C04B 35/50**(2006.01)i; **C07C 5/333**(2006.01)i; **C07C 11/167**(2006.01)i

According to International Patent Classification (IPC) or to both national classification and IPC

**B. FIELDS SEARCHED**

Minimum documentation searched (classification system followed by classification symbols)

B01J 37/08(2006.01); B01J 23/10(2006.01); B01J 23/22(2006.01); B01J 23/78(2006.01); B01J 23/889(2006.01); C01G 25/00(2006.01); C07C 5/48(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 세륨(cerium), 지르코니아(zirconia), 산화물(oxide), 부타디엔(butadiene), 아연(zinc)

**C. DOCUMENTS CONSIDERED TO BE RELEVANT**

| Category* | Citation of document, with indication, where appropriate, of the relevant passages | Relevant to claim No. |
|---|---|---|
| X | KR 10-2005-0042039 A (MAGNESIUM ELEKTRON LTD.) 04 May 2005 (2005-05-04)<br>See claims 11-16; and paragraphs [0026]-[0047]. | 1-9 |
| Y | | 10-13 |
| Y | KR 10-2033867 B1 (UOP LLC) 17 October 2019 (2019-10-17)<br>See claim 1; and paragraph [0023]. | 10-13 |
| A | KR 10-2030121 B1 (KOREA KUMHO PETROCHEMICAL CO., LTD.) 08 October 2019 (2019-10-08)<br>See entire document. | 1-13 |
| A | JP 2013-536066 A (SK INNOVATION CO., LTD.) 19 September 2013 (2013-09-19)<br>See entire document. | 1-13 |
| A | KR 10-2000-0011064 A (RHODIA CHIMIE) 25 February 2000 (2000-02-25)<br>See entire document. | 1-13 |

☐ Further documents are listed in the continuation of Box C.    ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **24 November 2021** | **25 November 2021** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

## INTERNATIONAL SEARCH REPORT
### Information on patent family members

International application No.

**PCT/KR2021/010534**

| Patent document cited in search report | | | Publication date (day/month/year) | Patent family member(s) | | | Publication date (day/month/year) |
|---|---|---|---|---|---|---|---|
| KR | 10-2005-0042039 | A | 04 May 2005 | AT | 303203 | T | 15 September 2005 |
| | | | | BR | 0213822 | A | 31 August 2004 |
| | | | | BR | 0213822 | B1 | 26 June 2012 |
| | | | | CA | 2465376 | A1 | 08 May 2003 |
| | | | | CA | 2465376 | C | 17 September 2013 |
| | | | | CN | 1582198 | A | 16 February 2005 |
| | | | | CN | 1582198 | C | 24 September 2008 |
| | | | | EP | 1444036 | A1 | 11 August 2004 |
| | | | | EP | 1444036 | B1 | 31 August 2005 |
| | | | | JP | 2003-137550 | A | 14 May 2003 |
| | | | | JP | 3946982 | B2 | 18 July 2007 |
| | | | | RU | 2004116469 | A | 10 April 2005 |
| | | | | RU | 2311956 | C2 | 10 December 2007 |
| | | | | US | 2006-0088463 | A1 | 27 April 2006 |
| | | | | US | 7431910 | B2 | 07 October 2008 |
| | | | | WO | 03-037506 | A1 | 08 May 2003 |
| | | | | ZA | 200402829 | B | 23 February 2005 |
| KR | 10-2033867 | B1 | 17 October 2019 | CA | 2996532 | A1 | 09 March 2017 |
| | | | | CA | 2996532 | C | 14 July 2020 |
| | | | | CN | 108698957 | A | 23 October 2018 |
| | | | | CN | 108698957 | B | 27 August 2021 |
| | | | | EP | 3341348 | A1 | 04 July 2018 |
| | | | | MX | 2018002452 | A | 18 June 2018 |
| | | | | RU | 2674762 | C1 | 13 December 2018 |
| | | | | US | 10526259 | B2 | 07 January 2020 |
| | | | | US | 2018-0327337 | A1 | 15 November 2018 |
| | | | | WO | 2017-040165 | A1 | 09 March 2017 |
| KR | 10-2030121 | B1 | 08 October 2019 | None | | | |
| JP | 2013-536066 | A | 19 September 2013 | CN | 103025425 | A | 03 April 2013 |
| | | | | CN | 103025425 | B | 20 January 2016 |
| | | | | EP | 2595749 | A2 | 29 May 2013 |
| | | | | EP | 2595749 | B1 | 19 September 2018 |
| | | | | JP | 5837062 | B2 | 24 December 2015 |
| | | | | KR | 10-1713328 | B1 | 08 March 2017 |
| | | | | KR | 10-2012-0009687 | A | 02 February 2012 |
| | | | | US | 2013-0158325 | A1 | 20 June 2013 |
| | | | | US | 9550174 | B2 | 24 January 2017 |
| | | | | WO | 2012-011659 | A2 | 26 January 2012 |
| | | | | WO | 2012-011659 | A3 | 19 April 2012 |
| KR | 10-2000-0011064 | A | 25 February 2000 | AT | 212322 | T | 15 February 2002 |
| | | | | AU | 1997-29658 | B2 | 09 March 2000 |
| | | | | AU | 716835 | B2 | 09 March 2000 |
| | | | | BR | 9709236 | A | 10 August 1999 |
| | | | | CA | 2255571 | A1 | 20 November 1997 |
| | | | | CA | 2255571 | C | 25 December 2001 |
| | | | | CN | 1177765 | C | 01 December 2004 |
| | | | | CN | 1222127 | A | 07 July 1999 |
| | | | | EP | 0906244 | A1 | 07 April 1999 |
| | | | | EP | 0906244 | B1 | 23 January 2002 |
| | | | | FR | 2748740 | A1 | 21 November 1997 |

Form PCT/ISA/210 (patent family annex) (July 2019)

**INTERNATIONAL SEARCH REPORT**
Information on patent family members

International application No.

**PCT/KR2021/010534**

| Patent document cited in search report | Publication date (day/month/year) | Patent family member(s) | | | Publication date (day/month/year) |
|---|---|---|---|---|---|
| | | FR | 2748740 | B1 | 21 August 1998 |
| | | JP | 2000-501061 | A | 02 February 2000 |
| | | JP | 2005-119949 | A | 12 May 2005 |
| | | JP | 3623517 | B2 | 23 February 2005 |
| | | JP | 4041106 | B2 | 30 January 2008 |
| | | NO | 322444 | B1 | 09 October 2006 |
| | | NO | 985313 | L | 15 January 1999 |
| | | US | 6228799 | B1 | 08 May 2001 |
| | | WO | 97-43214 | A1 | 20 November 1997 |
| | | ZA | 974025 | B | 16 January 1998 |

Form PCT/ISA/210 (patent family annex) (July 2019)

**REFERENCES CITED IN THE DESCRIPTION**

**Patent documents cited in the description**

• KR 1020200110340